# EUROPEAN PATENT APPLICATION

(11) **EP 1 034 747 A1**
(43) Date of publication of application: **13.09.2000**
(21) Application number: 00301745.6
(22) Date of filing: 03.03.2000
(51) Int. Cl.: A61B 18/14

(54) **Electrosurgery system and instrument**

(30) Priority: 05.03.1999 GB 9905211
(71) Applicant: GYRUS MEDICAL LIMITED, Cardiff CF3 0LT (GB)
(72) Inventor: Goble, Colin Charles Owen, South Glamorgan, CF64 1AT, Wales (GB); Amoah, Francis, St Mellons, Cardiff CF3 0LT (GB); Bedford, Mark, St Mellons, Cardiff CF3 0OQ, Wales (GB)
(74) Representative: Blatchford, William Michael

(57) **Abstract**

An electrosurgical instrument has a handpiece, an elongate shaft (22) mounted to the handpiece and including electrical supply conductors (24, 26) and, located at the distal end of the shaft, an active electrode (12) and a return electrode (14). Housed at the distal end of the shaft is a frequency up-converter in the form of a spark-gap device (24A, 26A, 36) which acts as a pulse generator operable in conjunction with a resonant output structure (12, 14, 16, 40, 42) to convert energy supplied via the electrical supply conductors at 1 MHz or lower to an alternating electrosurgical signal at a frequency of at least 500 MHz.

## Description

This invention relates to a unipolar radio frequency electrosurgical system having an electrode which is applied to tissue to be treated to produce an electrosurgical effect and a return conductor which provides for a capacitive return path between the tissue and the system. The invention also relates to an electrosurgical instrument for the system.

Such a system falls into a class of electrosurgical devices distinguished by requiring only one or more active electrodes to be in contact with or adjacent tissue being treated, whilst having one or more return conductors situated in a medium spaced from the surface of the treated tissue to provide a local current return from the tissue to the radio frequency (R.F.) energy source supplying power for the electrosurgical effect. The devices include so-called underwater electrode assemblies such as disclosed in WO 97/00647 and EP 0754437 , which provide for a local current return from the tissue through a conductive liquid medium such as saline to a return electrode. Such devices also include an instrument in which local current return is provided by means of a capacitive return path, as disclosed in WO 97/15237 which relates to a self-contained handheld instrument having a radio frequency generator housed in a conductive casing held by the surgeon during use of the instrument. The casing constitutes a capacitive element forming part of both direct and indirect capacitive return paths from the tissue to the R.F. source, whether directly through air or indirectly through the surgeon's body and surroundings.

The disadvantages associated with the above devices include the need to supply fluid to the site of the operation, and in the case of an instrument relying on capacitive return paths, the limitations of the generator and the variability of the return paths restricting operation to relatively low electrosurgical powers.

According to a first aspect of this invention, an electrosurgical system comprises an electrosurgical generator and an electrode assembly comprising an elongate shaft including a tubular sheath, at least one active electrode for feeding electrosurgical currents from the generator to tissue to be treated, and a return conductor set back from the active electrode, wherein the system is operable to deliver an alternating electrosurgical voltage between the active electrode and the return conductor at a frequency in excess of 100 MHz, and wherein the return electrode is electrically isolated from the sheath at the said frequency. Use of a frequency in excess of 100 MHz and isolation of the return electrode has the effect of reducing the electrical impedance of the return path in comparison with lower frequency devices and concentrating the dissipation of energy to a comparatively small region adjacent the active electrode. The effect is more pronounced at 300 MHz and upwards. Since the return conductor is located and dimensioned to provide a capacitive return path, it can be covered with an insulative layer. Isolation of the return conductor may be performed by means of a transformer located close to the conductor. The transformer may be very small at UHF frequencies, to the extent that it may fit within a shaft of no more than 5mm outside diameter.

In a particular preferred embodiment of the invention, the generator is operable to deliver an alternating voltage to the electrode assembly in a frequency range at least one order of magnitude lower in frequency than the predominant frequency used to obtain the electrosurgical effect by having a frequency up-converter as part of the electrode assembly. This converter may comprise a pulse generator such as a spark-gap breakdown device in conjunction with a resonant output circuit resonant at the electrosurgical frequency, which is typically in excess of 300MHz. At these ultra high frequencies (UHF), and especially above 1GHz, the components of the up-converter may be made small enough to fit within the instrument shaft adjacent the return conductor.

The spark gap device may simply comprise a pair of spark gap elements at the end of a coaxial feeder, the outer screen of which feeder is typically constituted by the tubular conductive sheath of the shaft. Sparking across the spark gap is achieved by building up energy in a discharge capacitor coupled to one of the spark gap feeder terminations so that, for instance, during each half cycle of the low frequency supply, energy builds up on the capacitor until the breakdown voltage of the gap is achieved, whereupon a very rapid discharge current pulse is produced having components which extends well into the UHF band. By coupling such current through, for instance, the transformer referred to above, to the resonant output circuit tuned to a required output frequency, much of the energy of the discharge pulse can be applied to the active electrode at the required frequency in excess of 100 MHz.

Typically, the return conductor is a dielectric-filled conductive tube which is insulated from the coaxial feeder outer screen, the active electrode being mounted in the dielectric core and having a tissue treatment portion extending outside the core at the extreme distal end of the shaft. The active electrode and its connections together define the resonant output circuit, having both lumped and distributed reactances.

In accordance with a second aspect of the invention, a unipolar electrosurgical instrument comprises the assembly of a handpiece, an elongate shaft including electrical supply conductors, the shaft being mounted to the handpiece, the combination of an active electrode and a return conductor both of which are located at an end of the shaft remote from the handpiece, and a frequency up-converter forming part of the assembly.

According to a third aspect of the invention a unipolar electrosurgical instrument comprises the assembly of a handpiece, an elongate shaft including electrical supply conductors, the shaft being mounted to the handpiece, and an active electrode and a return conductor both of which are located at an end of the shaft remote from the handpiece and arranged to receive electrosurgical power via the supply conductors, the return conductor comprising a capacitive element covered by an insulative layer.

The invention also includes an electrosurgical system comprising an electrosurgical radio frequency generator and an electrode assembly, the electrode assembly comprising: a handpiece; an elongate shaft having a proximal end mounted to the handpiece, and a pair of electrical supply conductors extending from the proximal end to the distal end of the shaft; and, at the said distal end, at least one active electrode and at least one return conductor; wherein the active electrode has an exposed tissue treatment portion projecting from the shaft, and the return conductor is set back from the said exposed active electrode portion to act as a capacitively coupled return element; wherein the system is arranged to deliver an alternating electrosurgical voltage between the active electrode and the return conductor at a frequency in excess of 100 MHz; and wherein the return conductor is electrically isolated from the supply conductors at the said frequency.

The invention will now be described by way of example with reference to the drawings in which:-
Figure 1 is a diagrammatic representation of an electrode assembly for illustrating the principle of operation of an electrosurgical instrument in accordance with the present invention;
Figure 2 is a longitudinally sectioned view of a distal end portion of an electrosurgical instrument shaft, including a spark gap frequency up-converter;
Figure 3 is an electrical block diagram of an electrosurgical system in accordance with the invention; and
Figure 4 is an electrical block diagram of an alternative electrosurgical system in accordance with the invention.
Figures 5A and 5B are, respectively, longitudinally and transversely sectioned views of the distal end portion of an instrument shaft containing a modified spark gap up-converter.

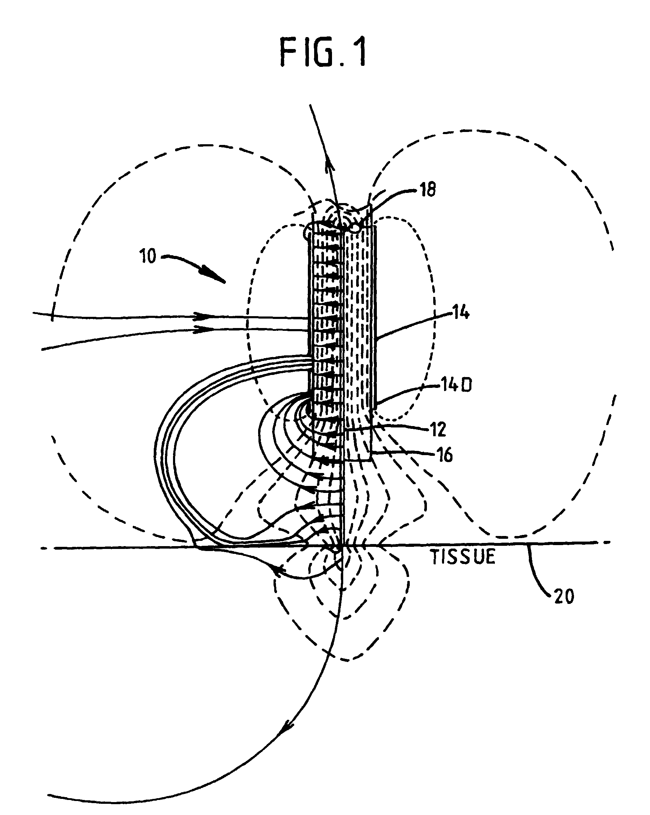

Referring to Figure 1, a coaxial electrode assembly 10 has a needle-like active electrode 12 which is axially mounted within an adjacent tubular return conductor 14 in the form of an elongate sleeve, the active electrode 12 projecting beyond a distal open end 14D of the return conductor 14. Where the active electrode is surrounded by the return conductor, it is embedded in a low-loss dielectric (e.g. a ceramic body 16 filling the interior of the return conductor 14 which thereby forms a sleeve, and having a relative dielectric constant higher than that of air.) The electrode assembly is unipolar in that the return conductor is close to but set back from the active electrode so that it is normally adjacent but spaced from tissue when the electrode is applied to the tissue, and so that it is capacitively coupled to the tissue.

It is to be understood that the assembly 10 constitutes the distal end portion of an instrument shaft, the remainder of the shaft not being shown in Figure 1. For the purposes of illustrating the principle of operation, then, the assembly 10 is shown as being energised from a voltage source 18 connected at a proximal end of the assembly, between the electrode 12 and return conductor 14. The active electrode 12 is shown with its distal end in contact with the surface of tissue 20 to be treated.

When a UHF alternating voltage is applied by the source 18, an electric field is set up, as shown by the dashed contours of constant potential in Figure 1. Currents flow along orthogonal paths as shown by the solid lines with arrows, the arrows corresponding to the direction of current flow with one polarity of the voltage source 18. The equal potential contours are spaced at equal increments of potential, although it should be noted that one additional contour, shown dotted, is included for clarity.

The electric field pattern is a result of the differing dielectric constants and conductivities of the tissue, the air surrounding the electrode assembly 10, and the dielectric body 16, with the biggest discontinuities occurring at the media interfaces. It is noticeable that a region of intense electric field is maintained near the active electrode 12 with the effect that collateral tissue damage is much reduced compared with many conventional devices. This is at least partly due to the high impedance of the tissue relative to the reactive impedance at UHF between the tissue and the return conductor 14. Indeed, the confinement of the intense electric field becomes greater, in general, the higher the frequency of the source and the smaller the size of the electrode assembly.

The relative bunching and curvature of the equal potential contours adjacent the tip of the active electrode 12 results in the greatest electrical current flow in this region, and, as a result of the dissipative nature of the tissue due to its resistive loss, the greatest power dissipation. In contrast, comparatively little dissipation occurs in the air and in the dielectric body 16 where, despite the contours being closely spaced, there is little dielectric loss or resistive loss.

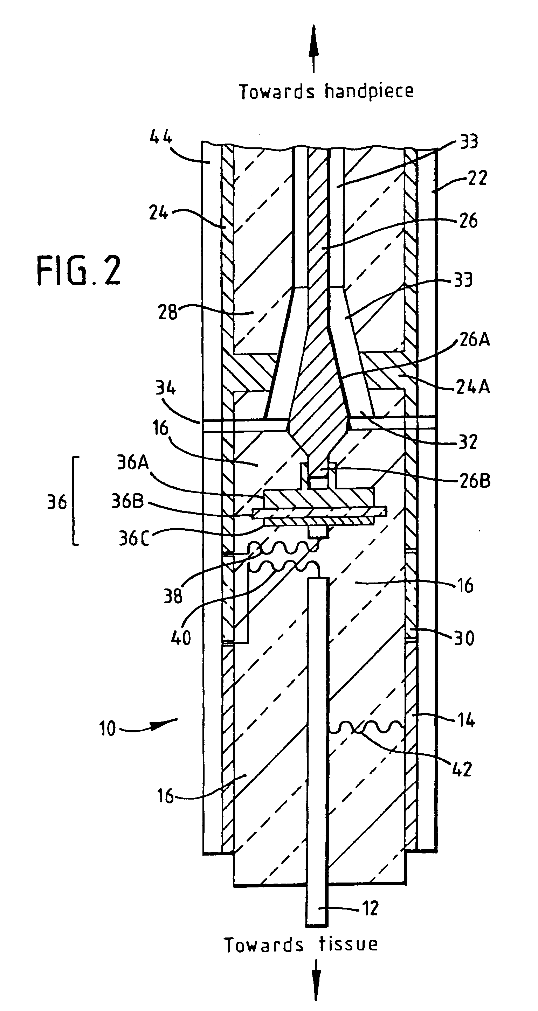

A preferred electrode assembly for putting into effect the principle described above with reference to Figure 1, will now be described with reference to Figure 2. Here, the electrode assembly 10 forms the distal end portion of an instrument shaft 22 such that the return electrode 14 is a conductive sleeve covering a portion of a low loss dielectric body 16 made of, for instance, a ceramic material. The active electrode 12 is embedded in a axial position in the dielectric body 16, as in Figure 1. Over the majority of its length, the shaft 22 constitutes a coaxial feeder having a metallic sheath or outer conductor 24 and an axial inner conductor 26, separated from the outer conductor 24 by a low loss dielectric material 28.

The return conductor 14 is of a similar diameter as the outer conductor 24 of the feeder, but is insulated from the latter by an annular insulative section 30, which may be a rib formed on the dielectric body 16. As shown in Figure 1, the return conductor 14 is set back from the tissue treatment portion of the active electrode 12, or at least its distal end, such that when the treatment portion is brought adjacent a tissue surface, the return conductor is spaced back from the surface. As a result, the return path from the tissue surface to the UHF source is by virtue of the capacitance between the tissue surface and the return conductor 14.

A notable feature of the instrument is a frequency up-converter within the shaft, in the form of a spark gap device formed by the combination of an enlarged end portion 26A of the inner conductor 26 and an inner annulus 24A forming part of the outer conductor 24 of the feeder adjacent its distal end. The gap 32 between these two spark gap elements 26A, 24A is annular and forms part of a passage 33 through which fluid may be pumped (via fluid inlet 34) as a means of controlling the breakdown voltage. The passage 33 delivers fluid, preferably gas, usually air, to the gap 32 to remove ionised gases thereby to decrease the recovery time of the gap. Other benefits include cooling the gap 32 and clearing vapour and smoke from the surgical site. Tapered formation of the inner conductor distal end portion 26A allows adjustment of the spark gap 32 by axial movement of the inner conductor 26.

Also forming part of the spark gap device is a discharge capacitor 36 within the ceramic dielectric body 16, constituted by a first capacitor plate 36A in which the distal end 26B of the inner conductor 26 is a sliding fit, a dielectric layer 36B, and a second capacitor plate 36C having a distal stud which is connected to the distal end of the outer conductor 24 by a conductive track 38 applied to the dielectric body 16. This track forms the primary winding of a transformer which has a parallel second winding formed by a second conductive track 40 connected between the proximal ends of the active electrode 12 and the return conductor 14.

A third conductive track 42 connecting the active electrode to the return conductor at an intermediate position between the two ends of the active electrode 12 acts as a shunt inductor for tuning purposes.

Both the outer conductor 24 of the feeder and the return conductor 14 are covered by an insulative sheath 44 which is continuous over the whole length of the shaft to the distal edge of the return conductor 14, with the exception of an aperture corresponding to gas inlet 34.

In use, the feeder 24, 26 is indirectly coupled to a radio frequency electrosurgical generator operating at a frequency between 10kHz and 1MHz, the coupling being via a transformer and/or other reactive elements which may be mounted in a handpiece of the instrument. The generator may be a proprietary generator operating at conventional electrosurgical frequencies, e.g. 350 kHz to 1 MHz. The level of the generator output voltage is preferably in the region of 300 volts peak or upwards, and is stepped up to a level in the order of 2000 V peak, this level being sufficient to cause arcing at the spark gap, the width of which is between 0.05mm and 5mm, and preferably between 0.2mm and 1mm. The voltage level at which arcing occurs depends not only on the width of the gap, but also on the substances within the gap. For instance, the gap may be filled with air, but other fluids can be introduced. During each half cycle of the generator output voltage the voltage across the spark gap 32 and capacitor 36 builds up and if it achieves a high enough level to cause ionisation of the gas in the spark gap, an abrupt transition from a high impedance to a low impedance state occurs causing an exceptionally rapid discharge of capacitor 36 and a high current transient in transformer primary 38. This transient has a wide range of frequency components, including at least one component at a resonant frequency defined by structure of the electrode assembly, especially the active electrode, its relationship with the return conductor 14, the dielectric constant of the dielectric body 16, and the inductances of the transformer secondary 40 the shunt inductance 42. Accordingly, a voltage pulse with a dominant UHF frequency component is generated at the active electrode distal end 12, where it is used to provide a localised electrosurgical effect, the return path being a capacitive path as described above with reference to Figure 1.

By controlling the applied low frequency voltage from the radio frequency electrosurgical generator, it can be arranged that spark gap breakdown occurs near the peak of the low frequency voltage waveform in order to produce the greatest transient output. This also minimises heating of the electrode and difficulty in extinguishing the arc, allowing the highest possible spark repetition rate and minimum spark duration. A 90 degree phase relationship between the zero-crossing of the low frequency excitation voltage and spark gap breakdown (the transient current edge) is optimal, and control of the low frequency output can be derived by monitoring the phase relationship between the current edge and the R.F. excitation voltage.

A gaseous supply via inlet 34 may be used to remove excess heat from the spark gap generator and allows re establishment of the ionisation potential of the gap. The flow rate necessary to achieve this is dependent on the spark repetition rate and other thermal dissipation conditions.

The energy within each spark discharge is dependent on applied voltage and the size of the discharge capacitor 36. It is not necessary to achieve a discharge with every half cycle of the applied low frequency voltage, and since the discharge capacitor can become part of a series-resonant circuit resonant at the generator frequency, it is possible to configure the gap and the series tuning components for a slow build-up of excitation energy due to ringing in the low frequency resonant circuit. When breakdown occurs, this built-up energy is dissipated, with the result that the current is thereafter insufficient to sustain the arc, the spark gap returns to a high impedance state, and the resonant circuit starts to ring again to begin another process of building up energy on the capacitor. Between individual sparks, the discharge capacitor forms part of the low frequency resonant circuit. When a spark occurs this capacitor forms part of the resonant structure of the electrode assembly, resonant at UHF.

This technique allows sparking at a relatively low frequency, typically 10 to 20kHz, with a comparatively high frequency source operating in the range 100 to 1000kHz. use of series resonance allows the voltage always to climb to a level sufficient to exceed the ionisation potential of the spark gap, such that breakdown occurs after several half cycles (typically between 5 and 100), which means that discharges are unlikely to occur at a strictly regular frequency with the result that no audible tone is generated by the sparking. If the generator has an operating frequency higher than the discharge frequency, the rate of rise of voltage across the gap tends to be quicker, with the result that the discharge threshold voltage and speed of the resulting discharge are increased.

It is possible to adjust the output frequency and tissue effect by simple mechanical adjustment of the components or their relationship. For example, the capacitance of the discharge capacitor may be made as small as 20pF and of a construction such that it can be varied by mechanical adjustment of one plate (not shown in Figure 2). This allows alteration of discharge energy and, therefore, electrical power delivered to the target site.

To summarise, it will be understood that the above-described arrangement provides a UHF frequency source (in this case a spark gap generator) in close proximity to the active electrode, but controlled and fired by a remote low frequency R.F. source. By provision of a capacitor having a dielectric material with low dielectric loss and sufficiently high dielectric constant that the capacitor can be made small enough to be placed within the electrode assembly, it has been possible to construct a tuned generator within the electrode shaft. The dielectric material is Rogers RO 4003, which is a woven-glass-reinforced ceramic-filled thermoset plastics material. The discharge capacitor is discharged by the spark gap into the primary winding of the transformer, which is air cored, and optionally tuned. Energy is magnetically coupled to the secondary winding in close proximity to the active electrode tip, the circuit being completed by capacitive coupling to the set back return conductor.

It is preferred that the UHF output is at a frequency greater than 500MHz, and possibly greater than 1GHz. This is much higher than the frequencies used in conventional unipolar systems and has the potential in some circumstances of creating radio frequency interference. However, such interference is minimised by the fact that frequency up-conversion occurs very close to the distal end of the shaft, with the result that UHF radiation from the coaxial feeder can be minimal. Minimising the distance between the UHF source and the active electrode also substantially reduces UHF losses in comparison with UHF power supplied via a cable.

One of the advantages of operation at the frequencies specified is that significant thermal margins can be produced in the tissue as a result of the capacitive coupling through inside the tissue, bearing in mind that capacitive coupling at these frequencies produces a relatively low output impedance and voltage gradient from electrode to tissue. Indeed, at frequencies in excess of 1GHz, the voltage gradient can be so shallow that arcing is prevented except at very high operating voltages. (Typically, low frequency voltages of the order of 300V peak cause arcing. Instead, at UHF, heating of the tissue occurs due to a dielectric heating effect.) This means that at the higher voltages and waveform crest factors used in conventional systems to overcome the impedance eschar build-up on electrode surfaces, the same output at these high frequencies produces a much more desirable "soft" coagulation with minimal charring irrespective of eschar build-up.

Location of the UHF source in close proximity to the active electrode minimises the antenna effect (i.e. interference) and the generated UHF energy is virtually all dissipated at the target site. It will be understood that it is not essential to the invention in its broadest sense that the frequency up-convertor is located adjacent the active electrode. Depending on circumstances, in particular the length of the instrument shaft, acceptable performance may be obtained by locating the up-converter/spark-gap device in a handpiece which mounts the proximal end of the instrument shaft, the low frequency source then being connected to the handpiece by a flexible coaxial cable in the conventional manner.

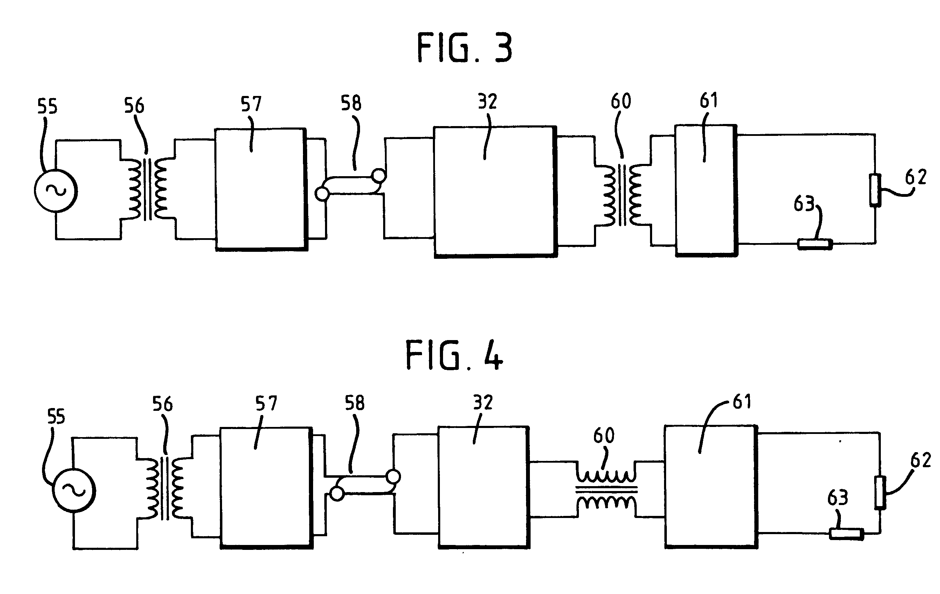

Preferred features of the system as a whole will now be described with reference to Figure 3. A low frequency R.F. generator 55 operating at between 10kHz and 1MHz feeds a voltage transformer 56 arranged to step up the output voltage of the source 55 to a level sufficient to cause arcing in the spark gap device described above. A reactive impedance stage 57, typically comprising a series inductance and parallel output capacitance, provides a high impedance barrier to UHF voltage components fed back from the spark gap device along the instrument shaft. The shaft itself constitutes a coaxial feeder or transmission line 58. This transmission line extends the length of the instrument shaft in the preferred embodiment.

The spark gap device 32 is represented as a block in Figure 3, which includes a frequency filter designed to transmit only high-order frequency components created by the spark gap device and to reject low frequency components from the low frequency energy source 55. Transformer 60, having primary and secondary windings 38 and 40 (see Figure 2) isolates the instrument shaft from the path between the tissue being treated and the return conductor. The reactances associated with the structure of the active electrode and return conductor are represented as a reactive impedance stage 61. This includes parasitic and lumped components arranged to achieve frequency selection and impedance matching between the spark gap high frequency voltage source and the load represented by the active electrode and the tissue being treated. The latter is represented as a resistive impedance 62, and the return path between the tissue and the return conductor is represented by an impedance 63 which, as described above, is primarily capacitive.

Transmission line element 58 can be considered as a combination of lumped components, specifically a shunt capacitor and a series inductance. These act both as a local bypass capacitor acting as part of the reservoir for the spark gap device and a current limiting inductor. It is possible to add a discrete shunt capacitance and series inductance to augment the distributed elements. Furthermore, a shunt inductance may be placed across the transmission line 58 at the proximal end to cause resonance with the inherent shunt capacitance. In this instance, the output from transformer 26 may be magnified over several cycles of the low frequency energy source.

With regard to the high frequency parts of the system, the high frequency transformer 60 is shown as relying on mutual inductance to achieve delivery of UHF voltage with isolation of the return conductor 14. The mutual inductance of the transformer windings may be enhanced by including a core material with a relative magnetic permeability high than 1, using, for instance, a nickel-zinc ferrite mix, or magnetic iron dust material.

Referring to Figure 4, in an alternative system a common mode arrangement is used for the high frequency transformer 60 to achieve high frequency isolation between distal and proximal ends of the instrument shaft. As in the embodiment of Figure 3, the reactive impedance stage 61 allows a narrow band of frequency components through to the distal end of the electrode, and an impedance transformation is achieved within this band between the source impedance represented by the transformer 60 and the nominal tissue impedance 62.

Selection of the frequency components to be transmitted may be performed by altering impedance stage 61 in order to alter tissue effects. For instance, coagulation may be enhanced by selecting higher frequency voltage components.

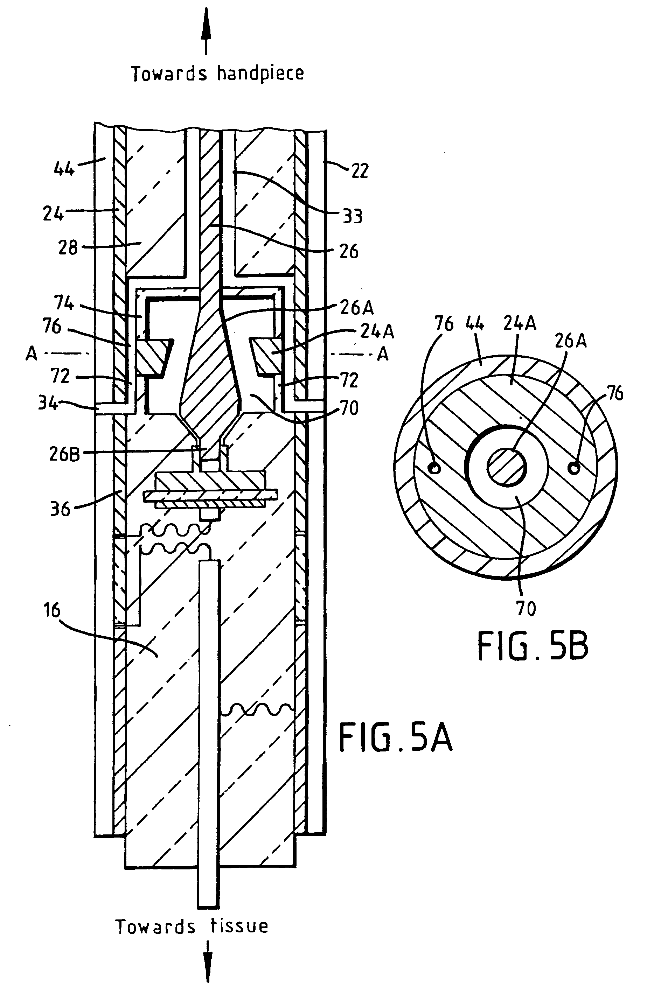

It has been mentioned above that the spark gap of the above-described instrument may be filled with a substance other than air. Whilst a selected fluid may be pumped through the passage 33 (see Figure 2) to remove ionised gases, for instance, the spark gap may be formed within a closed fluid-filled chamber. Referring to Figures 5A and 5B, a modified instrument has the annular spark gap 32 formed within a closed fluid-filled chamber or enclosure 70. One preferred fluid for chamber 70 is SF₆.

Both the tapered inner conductor distal end portion 26A and the inner surface of the inner annulus 24A (i.e. the spark gap terminals) are wholly within the chamber 70, the latter being defined by insulative walls 72 and 74 extending proximally from the dielectric body 16 surrounding the discharge capacitor 36 and distally from the dielectric insulative 28 of the feeder.

Inlet 34 and passage 33 are retained from the embodiment described with reference to Figure 2, to allow passage of cooling fluid through bores 76 in the annulus 24A. In viewing Figure 5B, it should be noted that this is a cross-section on the line AA in Figure 5A.

Providing a fluid dielectric in the spark gap in this way allows the spark gap breakdown voltage to be increased, yielding larger amplitude high frequency transient components.

## Claims

1. An electrosurgical system comprising an electrosurgical generator an electrode assembly, the electrode assembly comprising an elongate shaft including a tubular sheath at least one active electrode for feeding electrosurgical currents from the generator to tissue to be treated, and a return conductor set back from the active electrode, wherein the system is operable to deliver an alternating electrosurgical voltage between the active electrode and the return conductor at a frequency in excess of 300 MHz, and wherein the return electrode is electrically isolated from the sheath at the said frequency.

2. A system according to claim 1, wherein the return conductor is electrically isolated from the sheath by a transformer located adjacent the return conductor.

3. A system according to claim 1 or claim 2, wherein the generator is operable to deliver an alternating voltage to the electrode assembly in a frequency range below 1 MHz, and wherein the electrode assembly includes a frequency up-converter.

4. A system according to claim 3, wherein the frequency up-converter comprises a pulse generator and a resonant output circuit resonant at a frequency in excess of 100 MHz.

5. A system according to claim 4 operable to deliver an alternating electrosurgical voltage between the active electrode and the return conductor at a frequency in excess of 300 MHz, the resonant output circuit being resonant at the said frequency.

6. A system according to claim 4 or claim 5, wherein the pulse generator comprises a breakdown device.

7. A system according to claim 6, wherein the breakdown device is a spark gap device.

8. A system according to any of claims 3 to 7, wherein the up-converter is located adjacent the return conductor.

9. A system according to claim 6 or claim 7, wherein tubular sheath forms part of a coaxial feeder and the breakdown device is located at a distal end of the sheath.

10. A system according to claim 9, wherein the breakdown device is a spark gap shunting the coaxial feeder.

11. A system according to claim 9 or claim 10, wherein the frequency up-converter includes a discharge capacitor coupled to the breakdown device and to the active electrode.

12. A system according to claim 11, wherein the discharge capacitor is coupled to the active electrode by a transformer, the primary winding of which is connected in series with the capacitor.

13. A system according to claim 12, wherein the transformer has a secondary winding coupled between the active electrode and the return conductor.

14. A system according to any of claims 8 to 13, wherein the active electrode comprises a rod having one end portion coupled to the frequency up-converter and an exposed distal end tissue treatment portion, the electrode assembly further comprising a shunt inductor connecting the active electrode to the return conductor at a location spaced from the two end portions of the active electrode, the rod and the shunt inductor forming part of the said resonant output circuit.

15. A system according to any preceding claim, wherein at least the major part of the outer surface of the return conductor is covered by an electrically insulative layer.

16. A system according to claim 15, wherein the return conductor is a elongate tubular conductor and the insulative layer extends from one end of the tubular conductor to the other.

17. A unipolar electrosurgical instrument comprising the assembly of:
a handpiece,
an elongate shaft including electrical supply conductors, the shaft being mounted to the handpiece,
an active electrode and a return conductor, both of which are located at an end of the shaft remote from the handpiece, and
a frequency up-converter.

18. An instrument according to claim 17, wherein the up-converter is located at the end of the shaft remote from the handpiece.

19. An instrument according to claim 17 or claim 18, wherein the up-converter comprises a spark gap device.

20. A system according to claim 17, wherein the electrode assembly includes a passage for the delivery of cooling fluid to the up-converter.

21. A system according to claim 20, wherein the up-converter comprises a spark gap device, and the fluid passage is arranged to deliver fluid to a spark gap forming part of the device thereby to control the ionisation voltage of the device.

22. A system according to claim 17, wherein up-converter includes a discharge capacitor, and the system is arranged such that the capacitor is excited resonantly by the generator output signal whereby the energy stored in the capacitor increases cycle by cycle until a discharge potential is reached.

23. A system according to claim 22, wherein the frequency of the generator output signal is between 100 Hz and 1 MHz.

24. A system according to claim 22 or claim 23, wherein the discharge capacitor forms part of a series tuned excitation circuit.

25. A system according to claim 22 or claim 23, wherein the discharge capacitor forms part of a parallel tuned excitation circuit.

26. A system according to any of claims 22 to 25, arranged such that the discharge capacitor reaches a discharge potential over no less than five half-cycles of the generator output signal.

27. A unipolar electrosurgical instrument comprising the assembly of:
a handpiece,
an elongate shaft including electrical supply conductors, the shaft being mounted to the handpiece, and
an active electrode and a return conductor both of which are located at an end of the shaft remote from the handpiece and arranged to receive electrosurgical power via the supply conductors, the return conductor comprising a capacitive element covered by an insulative layer, the active and return electrodes forming part of a resonant structure resonant at a frequency in excess of 100 MHz.

28. An instrument according to claim 27, wherein the return conductor comprises an elongate tubular conductor and the insulative layer extends from one end of the tubular conductor to the other.

29. An electrosurgical system comprising an electrosurgical radio frequency generator and an electrode assembly, the electrode assembly comprising:
a handpiece;
an elongate shaft having a proximal end mounted to the handpiece, a distal end, and a pair of electrical supply conductors extending from the proximal end to the distal end; and
at the said distal end of the shaft, at least one active electrode and at least one return conductor;
wherein the active electrode has an exposed tissue treatment portion projecting from the shaft, and the return conductor is set back from the said exposed active electrode portion to act as a capacitive return element;
wherein the system is arranged to deliver an alternating electrosurgical voltage between the active electrode and the return conductor at a frequency in excess of 300 MHz; and
wherein the return conductor is electrically isolated from the supply conductors at the said frequency.

30. a system according to claim 29, wherein the return conductor is covered by an insulative layer at least over a distal end portion.

31. An electrosurgical system comprising at least an electrosurgical generator and an electrode assembly, wherein the electrode assembly has a distal tip for application of electrosurgical current to tissue, a return element located so as to provide a return path for the electrosurgical current by capacitive coupling between the return element and the tissue, and a frequency converter for converting the output of the electrosurgical generator to a frequency which is at least an order of magnitude higher in frequency than the output signal produced by the generator.

32. A system according to claim 31, wherein the electrode assembly comprises a handpiece, and mounted to the handpiece, an instrument shaft which carries at its distal end (a) an active electrode forming the distal tip and (b) the return element in close proximity to the active electrode tip.

33. A system according to claim 32, wherein the frequency converter is located in the shaft, adjacent the active electrode and the return element.

34. A system according to claim 32, wherein the converter is located in the handpiece.

35. A system according to claim 31, wherein the converter comprises a pulse generator with an output rise time no greater than 20 nanoseconds, and wherein the electrosurgical generator has an output frequency greater than 10 kHz.

36. An electrosurgical system comprising at least an electrosurgical generator and an electrode assembly, wherein the electrode assembly comprises an electrode assembly shaft with an active electrode at its distal end and a return electrode set back from the active electrode for capacitive coupling to tissue to be treated, and wherein the electrode assembly further comprises a frequency converter for converting the output of the electrosurgical generator to a higher frequency, the generator being configured such that its output voltage is controlled to the minimum necessary to operate the frequency converter.

37. A system according to claim 36, wherein the frequency converter is a spark gap device.

38. A system according to claim 36 or claim 37, wherein the output voltage of the generator is controlled in response to the phase difference between the rising edge of current supplied from the generator to the electrode assembly and the phase of the generator output voltage.

39. A system according to claim 38, wherein the output voltage is controlled such that the said phase difference is substantially 90°.

40. An electrosurgical system comprising at least an electrosurgical generator and an electrode assembly, wherein the electrode assembly includes a frequency converter for converting the frequency of the generator output signal to a higher frequency, the frequency converter including a discharge capacitor, the system further comprising means operable to adjust the output power of the generator by varying the capacitance of the discharge capacitor.

41. An electrosurgical system having an electrosurgical generator and electrode assembly for introduction into a target region for tissue treatment, a method comprising converting the frequency of an electrosurgical signal supplied to the electrode assembly by the generator, the conversion being performed using a pulse generator to yield an output signal in the electrode assembly having a frequency at least ten times the frequency of the electrosurgical signal from the generator.

42. A method according to claim 41, wherein the frequency up-conversion is performed by discharging a spark gap device and selecting a high frequency component of a resulting current pulse.

43. A method according to claim 41 or claim 42, wherein the up-converter includes a discharge capacitor and the method comprises charging the discharge capacitor cycle-by-cycle of the generator electrosurgical signal to increase the discharge potential on the capacitor, and wherein the discharge repetition rate of the up-converter is substantially lower than the frequency of the electrosurgical signal.

44. A method according to any of claims 41 to 43, wherein the discharge potential is maximised by cycle-by-cycle charging of a discharge capacitor, the discharge repetition rate being substantially lower than the generator output frequency.
